(19) Europäisches Patentamt
European Patent Office
Office européen des brevets

(11) EP 2 671 498 B1

(12) EUROPEAN PATENT SPECIFICATION

(45) Date of publication and mention
of the grant of the patent:
**18.10.2017 Bulletin 2017/42**

(51) Int Cl.:
*A61B 1/00* (2006.01)    *A61B 1/04* (2006.01)
*G02B 23/24* (2006.01)    *H04N 5/225* (2006.01)
*H04N 5/232* (2006.01)    *H04N 9/04* (2006.01)

(21) Application number: **12827919.7**

(22) Date of filing: **24.08.2012**

(86) International application number:
**PCT/JP2012/071496**

(87) International publication number:
**WO 2013/031701 (07.03.2013 Gazette 2013/10)**

(54) **ENDOSCOPE DEVICE**

ENDOSKOPVORRICHTUNG

DISPOSITIF D'ENDOSCOPE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.08.2011 JP 2011185127
26.08.2011 JP 2011185128**

(43) Date of publication of application:
**11.12.2013 Bulletin 2013/50**

(73) Proprietor: **Olympus Corporation
Tokyo 192-8507 (JP)**

(72) Inventors:
• **HASHIMOTO, Susumu
Hachioji-shi,
Tokyo 192-8507 (JP)**

• **KANEKO, Kazuma
Hachioji-shi,
Tokyo 192-8507 (JP)**

(74) Representative: **Gunzelmann, Rainer
Wuesthoff & Wuesthoff
Patentanwälte PartG mbB
Schweigerstraße 2
81541 München (DE)**

(56) References cited:
**EP-A1- 2 301 415        WO-A1-2010/131620
US-A1- 2011 069 199**

**Description**

Technical Field

The present invention relates to an endoscope apparatus that is favorable for narrow band observation.

Background Art

An endoscope for medical use needs a light source apparatus that illuminates an inside of a body, since a site to be observed is an interior of a living body. Illuminating light generated by the light source apparatus is irradiated to tissue to be observed from a distal end portion at which an image pickup section is present via a light guide that is inserted through an insertion portion of an endoscope.

As observation by an endoscope, normal light observation (white light imaging: WLI) using a visible light is widely performed. In a frame-sequential endoscope apparatus, a light source of a white color light is transmitted through a rotation filter, and thereby, tissue in a body cavity is sequentially irradiated with illuminating lights of three colors that are R, G and B. Subsequently, the reflected light images corresponding to the illuminating lights of the three colors that are R, G and B are acquired in a time division manner, and a color image for performing normal light observation is generated from the respective reflected light images.

Further, various kinds of special light observation using wavelength characteristics of illuminating lights have been conventionally performed. For example, International Publication No. 2010/131620 (hereinafter, document 1) discloses an apparatus according to the preamble of claim 1 and comprising a frame-sequential type image pickup apparatus for performing narrow band observation (narrow band imaging: NBI) as special light observation. In narrow band observation, in order to observe blood vessels with high contrast, attention is paid to use of a light that has both the advantages of being strongly absorbed by blood, and being strongly reflected and scattered at a mucosal epithelium, and living tissue is sequentially irradiated with a blue color narrow band light and a green color narrow band light, whereby contrast of capillary vessels in a mucosal epithelium and thick vessels in a deep part is emphasized and displayed.

In the invention of document 1, a narrow band light G of a green color and two narrow band lights B1 and B2 of a blue color are configured to be capable of being irradiated sequentially. In the image pickup apparatus of document 1, narrow band observation is performed by using the narrow band observation image which is created from the reflected light images (narrow band images) corresponding to the narrow band lights G, B1 and B2.

However, the invention of document 1 is configured to determine a synthesis ratio based on an average brightness of a picked-up image. Since an average brightness of a picked-up image is not necessarily the same as a brightness sensed by a person, the invention of document 1 had a problem that light adjustment control corresponding to the sense of an operator is not performed. In addition, since the living tissue is irradiated with the narrow band lights B1 and B2 temporarily shifted, synthesizing the images based on the narrow band lights B1 and B2 caused a problem that blurring can occur in the synthesized image in some cases.

The present invention has an objective to provide an endoscope apparatus that allows for light adjustment control corresponding to the sense of an operator while suppressing image quality degradation.

Disclosure of Invention

Means for Solving the Problem

An endoscope apparatus according to one aspect of the present invention includes the features of claim 1.

Brief Description of the Drawings

Fig. 1 is a block diagram showing an endoscope apparatus according to one embodiment of the present invention;
Fig. 2 is an explanatory view showing one example of a rotation filter 33;
Fig. 3 is a block diagram showing a specific configuration of a brightness calculation processing section 44;
Fig. 4 is a block diagram showing a second embodiment of the present invention;
Fig. 5 is a block diagram showing an endoscope apparatus according to a first embodiment of the present invention;
Fig. 6 is an explanatory diagram for explaining brightness detection processing of picked-up images based on respective illuminating lights in the brightness calculation processing section 44 in Fig. 1;
Fig. 7 is an explanatory diagram for explaining the brightness detection processing of the picked-up images based on the respective illuminating lights in the brightness calculation processing section 44 in Fig. 1;

Fig. 8 is a graph for explaining a weight that changes in accordance with a mode;
Fig. 9 is a graph for explaining the weight that changes in accordance with the mode;
Fig. 10 is a block diagram showing the second embodiment of the present invention;
Fig. 11 is a block diagram showing an ordinary circuit that performs white balance adjustment;
Fig. 12 is a timing chart showing a state of performing conversion from interlace to progressive; and
Fig. 13 is an explanatory view for explaining an operation of a median filter.

Best Mode for Carrying Out the Invention

Hereinafter, embodiments of the present invention will be described with reference to the drawings.

(First Embodiment)

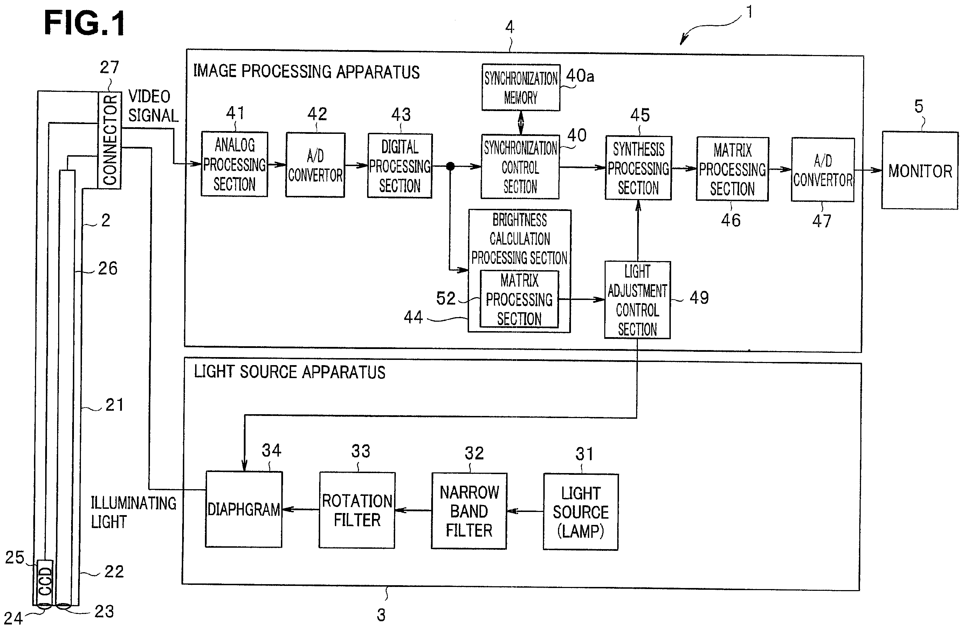

Fig. 1 is a block diagram showing an endoscope apparatus according to a first embodiment of the present invention.

As shown in Fig. 1, an endoscope apparatus 1 includes an endoscope 2 for observing an interior of a living body as a subject, a light source apparatus 3 that irradiates an illuminating light of a narrow band to perform observation of the interior of the living body, and an image processing apparatus 4 that performs signal processing to an image pickup signal of an image picked up under the illuminating light of the narrow band. A narrow band image generated by the image processing apparatus 4 is supplied to a monitor 5. As the monitor 5, an ordinary color monitor can be adopted. Namely, the monitor 5 includes an RGB input terminal (not illustrated), and signals of an R image, a G image and a B image are supplied to the RGB input terminal, whereby color display is performed.

The endoscope 2 has a flexible insertion portion 21 having such an outside diameter that can be inserted into a body cavity, and, a light guide fiber 26 that is for guiding a light irradiated from the light source apparatus 3, and is configured by a quartz fiber or the like is inserted through an interior of the insertion portion 21. One end of the light guide fiber 26 is connected to a connector 27 that is detachably connected to the light source apparatus 3. The other end of the light guide fiber 26 is disposed in a vicinity of an illumination lens 23 provided at a distal end portion 22 at a distal end of the insertion portion 21. Note that the connector 27 is connected to the light source apparatus 3 and is also connected to the image processing apparatus 4 which will be described later.

The illuminating light from the light source apparatus 3 is guided to the distal end portion 22 of the insertion portion 21 by the light guide fiber 26, and is diffused by the illumination lens 23 to be irradiated to a subject. Further, the distal end portion 22 is provided with an objective lens 24 for forming an optical image of a subject by a return light from the subject, and a CCD (charge coupled device) 25 as an image pickup device that is disposed in an image forming position thereof. The CCD 25 which configures image pickup means is driven by a CCD driving circuit not illustrated that is provided in the image processing apparatus 4 (not illustrated), picks up an image of a subject, converts an optical image of the subject which is picked up into a video signal, and outputs the video signal to the image processing apparatus 4.

The light source apparatus 3 includes a light source 31 that is configured by a xenon lamp or the like. The light source 31 emits a light of a wavelength band that is close to a white color light. On an irradiation optical path of the light source 31, a narrow band filter 32, a rotation filter 33 and a diaphragm 34 are placed.

The narrow band filter 32 makes a band of a light that is emitted from the light source 31 a narrow band, and emits the light to the rotation filter 33. The rotation filter 33 restricts the band of the light which passes through the narrow band filter 32 to a wavelength band that is necessary for narrow band observation. The diaphragm 34 restricts the light quantity of the light which passes through the rotation filter 33, and thereby adjusts the light quantity. The diaphragm 34 is configured to have a diaphragm closing amount controlled by a light adjustment control section 49 which will be described later.

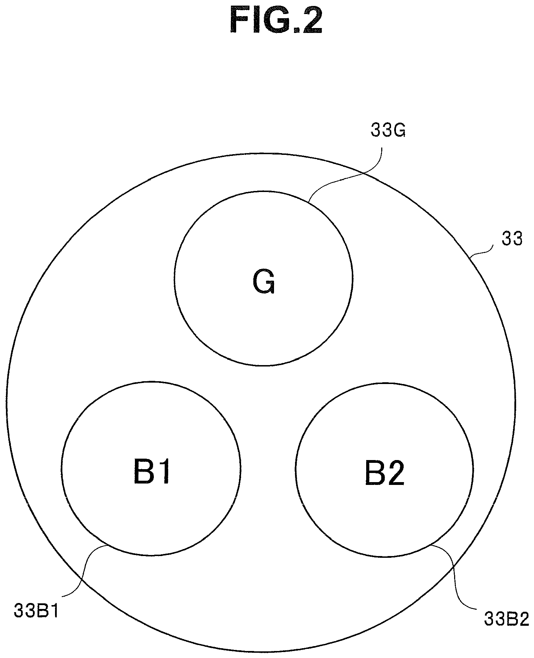

Fig. 2 is an explanatory view showing one example of the rotation filter 33.

The rotation filter 33 is in a disk shape, and is provided with three openings at equal angles in a circumferential direction, and filters 33G, 33B1 and 33B2 are respectively attached to the three openings. The filter 33G has a wavelength band of green (G) as a transmission band, and the filters 33B1 and 33B2 have a wavelength band of blue (B) as a transmission band.

By the narrow band filter 32 and the rotation filter 33, a G illuminating light of a narrow band of, for example, 530 to 550 nm with 540 nm as a center is transmitted from the filter 33G, a B illuminating light (hereinafter, called a B1 illuminating light) of a narrow band of, for example, 400 to 430 nm with 415 nm as a center is transmitted from the filter 33B1, and a B illuminating light (hereinafter, called a B2 illuminating light) of a narrow band of, for example, 400 to 430 nm with 415 nm as a center is transmitted from the filter 33B2 similarly to the filter 33B1. As above, the B1 illuminating light and the B2 illuminating light of the narrow band which are transmitted through the filters 33B1 and 33B2 are in the same wavelength band.

The rotation filter 33 has a center attached to a rotation shaft of a rotation motor not illustrated, and is rotationally driven. An encoder not illustrated is attached to the rotation shaft or the like of the rotation motor, and rotation of the rotation motor, that is, rotation of the rotation filter 33 is detectable by the encoder. The image processing apparatus 4 which will be described later controls the rotation of the rotation motor so that a rotation speed of the rotation filter 33 becomes constant (not illustrated).

As above, in the present embodiment, image pickup to a subject is performed by using the illuminating light of a narrow band from the light source apparatus 3. Therefore, as compared with the case of using an illuminating light of a wide band which is normally used widely, the illuminating light quantity sometimes tends to be insufficient. In particular, from the optical transmission characteristics by the light guide fiber 26, transmission loss at the short wavelength B side tends to be large, and an exit light quantity of the B illuminating light in the case of exiting as an illuminating light from the illumination lens 23 of the distal end portion 22 tends to be small.

Accordingly, in the present embodiment, the two filters 33B1 and 33B2 with the same transmission characteristics are disposed in the circumferential direction of the rotation filter 33, the same site of the subject to be the object to be observed is irradiated with the B illuminating light twice each time the rotation filter 33 is rotated once with use of the two filters 33B1 and 33B2, and image pickup based on the B illuminating light is performed twice by the return lights. For example, the rotation filter 33 is rotated once in a frame period of 1.5, and image pickup by the B illuminating light is performed twice. Subsequently, images picked up twice are synthesized, whereby the brightness of the picked-up image (B picked-up image) based on the B illuminating light is enhanced.

Note that the example in which image pickup by the G illuminating light is performed once, and image pickup by the B illuminating light is performed twice in the frame period of 1.5 is described, but the period and the number of times of performing image pickup by the narrow band lights of the respective colors can be properly set.

However, a B1 picked-up image based on the return light of the B1 illuminating light of the narrow band, and a B2 picked-up image based on the return light of the B2 illuminating light are images which are temporarily shifted, and the image quality is liable to be degraded by the images being synthesized. Accordingly, in the present embodiment, synthesis is not performed when an picked-up image with a sufficient brightness can be obtained by only the light of one of the B1 illuminating light and the B2 illuminating light of the narrow band. Further, when a picked-up image with a sufficient brightness cannot be obtained with only the light of one of the B1 illumination light and the B2 illumination light of the narrow band, a picked-up image based on the B illuminating light of the other one is synthesized in accordance with the brightness, and thereby, the picked-up image with the sufficient brightness is obtained while image degradation is suppressed.

In this case, in the present embodiment, the image processing apparatus 4 is configured to perform brightness control corresponding to a sense of a surgeon by finding the brightness of the picked-up image by color conversion matrix processing.

The image processing apparatus 4 has an analog processing section 41. The analog processing section 41 applies predetermined analog signal processing such as amplification processing to a video signal from the endoscope 2, and outputs the video signal to an A/D convertor 42. The A/D convertor 42 converts output of the analog processing section 41 into a digital signal, and thereafter, outputs the digital signal to a digital processing section 43.

The CCD 25 of the endoscope 2 outputs a G picked-up image based on a return light of the G illuminating light as a G signal, outputs the B1 picked-up image based on the return light of the B1 illuminating light as a B1 signal, and outputs the B2 picked-up image based on the return light of the B2 illuminating light as a B2 signal. The video signal from the endoscope 2 includes the G signal, the B1 signal and the B2 signal. A synchronization control section 40 causes a synchronization memory 40a that stores an R image, a G image and a B image to store the G signal, the B1 signal and the B2 signal. Note that in the synchronization memory 40a, for example, 30 frames of each of the G signal, the B1 signal and the B2 signal are stored. The signals of G, B1 and B2 are read from the synchronization memory 40a so that a color shift becomes minimum.

A brightness calculation processing section 44 calculates a brightness of the picked-up image sequentially at each 0.5 frame based on the G signal, the B1 signal and the B2 signal, before the G signal, the B1 signal and the B2 signal are recorded in the synchronization memory 40a. In the present embodiment, in order to evaluate the brightness of the picked-up image in the brightness in the case in which the picked-up image is actually displayed on the monitor 5, matrix processing similar to matrix processing that is performed on the occasion of display onto the monitor 5 is carried out, and the brightness is obtained. Further, in the present embodiment, illumination by the G illuminating light and the B1 illuminating light is set as master illumination that is always used in image pickup, and illumination by the G illuminating light and the B2 illuminating light is set as slave illumination that is used in an auxiliary manner when the brightness of the image is low. In order to find the brightness of the picked-up image by the master illumination, the brightness calculation processing section 44 finds a luminance Y1 by the master illumination by using matrix processing by a matrix processing section 52 based on the G signal and the B1 signal. Further, in order to find the brightness of the picked-up image by the slave illumination, the brightness calculation processing section 44 finds a luminance Y2 by the slave illumination by using the matrix processing by the matrix processing section 52 based on the G signal and the B2 signal.

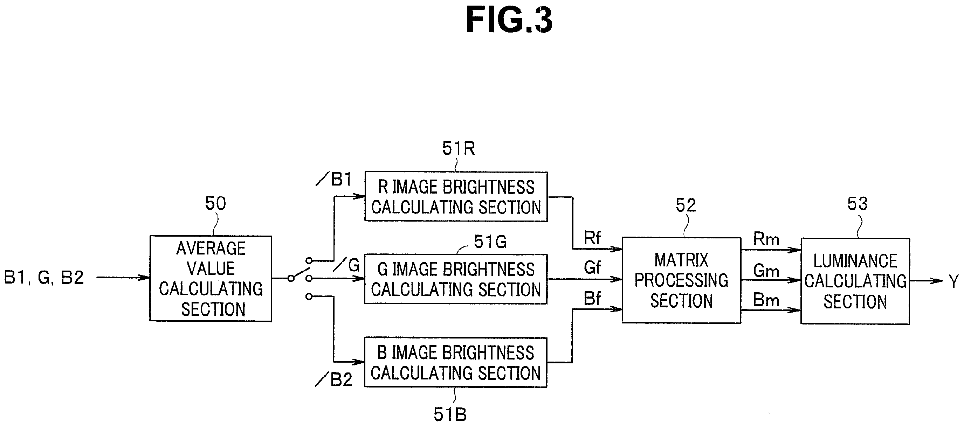

Fig. 3 is a block diagram showing a specific configuration of the brightness calculation processing section 44. An average value calculating section 50 calculates /the B1 signal, /the G signal, /the B2 signal that is an average value of the signals corresponding to the R image, the G image and the B image. An R image brightness calculating section 51R, a G image brightness calculating section 51G, a B image brightness calculating section 51B respectively find brightnesses by /the B1 signal, /the G signal, /the B2 signal. Note that in the present embodiment, the B1 signal is used as the signal of the R image, the G signal is used as the signal of the G image, and the B2 signal is used as the signal of the B image. The brightness calculating sections 51R, 51G and 51B retain /the B1 signal, /the G signal, /the B2 signal, and thereafter, output /the B1 signal, /the G signal, /the B2 signal to the matrix processing section 52 as Rf, Gf and Bf.

The matrix processing section 52 performs color conversion of the inputted signals of the R, G and B images by matrix calculation of the following equation (1). In this case, the matrix processing section 52 performs matrix processing for each of the master illumination and the slave illumination by the matrix calculation of the following equation (1). Note that $\alpha$, $\beta$ and $\gamma$ of equation (1) are matrix coefficients. From the matrix processing section 52, outputs Rm, Gm and Bm that are obtained by the matrix processing to the master illumination and the matrix processing to the slave illumination are supplied to a luminance calculating section 53. Note that in the present embodiment, the G signal as the output Rm, the B signal as the output Gm and the B signal as the output Bm are supplied to the luminance calculating section 53.

$$\begin{pmatrix} Rm \\ Gm \\ Bm \end{pmatrix} = \begin{pmatrix} 0 & \alpha & 0 \\ 0 & 0 & \beta \\ 0 & 0 & \gamma \end{pmatrix} \begin{pmatrix} Rf \\ Gf \\ Bf \end{pmatrix} \qquad \cdots (1)$$

The luminance calculating section 53 finds luminance by calculation to the signal Rm (G signal), the signal Gm (B signal) and the signal Bm (B signal) which are inputted. Namely, the luminance calculating section 53 obtains the luminance Y1 by the master illumination by, for example, calculation of the following equation (2).

$$Y1 = 0.3Rm + 0.59Gm + 0.11Bm = 0.3(\alpha \cdot /G) + 0.59\,(\beta \cdot /B1) + 0.11(\gamma \cdot /B1) \ldots \quad (2)$$

Further, the luminance calculating section 53 obtains the luminance Y2 by the slave illumination by, for example, calculation of the following equation (3) with a level of the B2 signal which is inputted in the luminance calculating section 53 set as B2.

$$Y2 = 0.3Rm + 0.59Gm + 0.11Bm = 0.3(\alpha \cdot /G) + 0.59(\beta \cdot /B2) + 0.11(\gamma \cdot /B2) \ldots \quad (3)$$

Here, G of the above described equation (2) and equation (3) satisfies G = Rm = $\alpha$Gf. The $\alpha$ is the same as $\alpha$ of equation (6) that will be described later. According to equation (5) that will be described later, Gt = (1+a)G is satisfied, and when a=1, Gt = 2G is satisfied. Accordingly, it is important to add 1/2 to $\alpha$.

The brightness calculation processing section 44 outputs the luminances Y1, Y2 and $\Delta$Y1 which are found to the light adjustment control section 49 as information of the brightness. The light adjustment control section 49 obtains a difference (Ys-Y1) = $\Delta$Y1 between a brightness (luminance) Ys as a target and the luminance Y1. In a case of $\Delta$Y1 $\leq$ 0, the brightness as the target is obtained by only the master illumination, and therefore, the light adjustment control section 49 sets a synthesis ratio (coefficient) a of the picked-up image by the slave illumination to be zero. Further, in a case of $\Delta$Y1 > 0, the light adjustment control section 49 finds the synthesis ratio a by the following equation (4). The light adjustment control section 49 outputs the found synthesis ratio a to a synthesis processing section 45.

$$\Delta Y1 = Y2 \times a \qquad\qquad a = \Delta Y1/Y2 \qquad\qquad \ldots (4)$$

In the narrow band observation, the picked-up images based on the respective return lights of the G illuminating light and the B illuminating light of the narrow band are used. The matrix processing section 46 which will be described later generates signal components of the RGB image from the G picked-up image and the B picked-up image which are obtained by image pickup using the narrow band lights by the matrix processing (color conversion matrix processing).

Matrix processing in the matrix processing section 52 of the brightness calculation processing section 44 is processing similar to the matrix processing in the matrix processing section 46. Namely, the matrix calculation by the brightness calculation processing section 44 is for obtaining the signals corresponding to R, G and B inputs of the monitor 5, the luminance which is obtained by the brightness calculation processing section 44 corresponds to the luminance of the image which is displayed on the monitor, and corresponds to the brightness of the image which is felt when the surgeon observes the monitor 5.

Note that the coefficients of the above described equation (2) and equation (3) in the brightness calculation processing section 44 are obviously changeable in response to a color tone desired as the narrow band observation image which is displayed on the monitor 5.

Further, the light adjustment control section 49 controls the diaphragm 34 so as to obtain a brightness to be a target, based on information of the brightness which is inputted. For example, when the brightness of the picked-up image is a target value or more, the light adjustment control section 49 outputs a light adjustment signal for reducing an opening amount of the diaphragm 34, and when the brightness of the picked-up image is less than the target value, the light adjustment control section 49 outputs a light adjustment signal for opening the diaphragm 34.

In the present embodiment, the synthesis processing section 45 synthesizes the picked-up image by the master illumination and the picked-up image by the slave illumination based on the synthesis ratio a. Namely, when the synthesis ratio a is zero, the synthesis processing section 45 outputs the picked-up image by the master illumination, that is, the signal using only the G signal and the B1 signal to the matrix processing section 46 from the digital processing section 43. Further, when the synthesis ratio a is not zero, the synthesis processing section 45 synthesizes the picked-up image by the slave illumination, that is, the signal which is obtained by the G signal and the B2 signal and the synthesis ratio a, and the signal based on the picked-up image by the master illumination.

Further, for example, when the synthesis ratio a becomes one because the brightness of the G illuminating light is extremely higher than the B illuminating light, electrically excessive multiplication is not present and increase of noise can be suppressed. The following equation (5) shows the synthesized signal by a. Note that Rin, Gin and Bin of equation (5) respectively represent inputs of the R image, the G image and the B image, and are respectively B2, G and B1 in the narrow band observation in the present embodiment. Further, Rt, Gt and Bt of equation (5) represent outputs of the R image, the G image and the B image of the synthesized signal. Note that in equation (5), the output of the R image is B2, but the output of the R image which is supplied to the monitor 5 that will be described later is substantially zero by matrix processing.

$$\begin{pmatrix} Rt \\ Gt \\ Bt \end{pmatrix} = \begin{pmatrix} 1 & 0 & 0 \\ 0 & 1+a & 0 \\ a & 0 & 1 \end{pmatrix} \begin{pmatrix} Rin \\ Gin \\ Bin \end{pmatrix} = \begin{pmatrix} 1 & 0 & 0 \\ 0 & 1+a & 0 \\ a & 0 & 1 \end{pmatrix} \begin{pmatrix} B2 \\ G \\ B1 \end{pmatrix}$$

$$= \begin{pmatrix} B2 \\ (1+a)G \\ a \cdot B2 + B1 \end{pmatrix} \quad \cdots (5)$$

The synthesis processing section 45 finds the synthesized signal by the calculation of equation (5), for example, and outputs the found synthesized signal to the matrix processing section 46. The matrix processing section 46 obtains signals corresponding to the RGB inputs of the monitor 5 by the matrix processing. The following equation (6) shows one example of the matrix processing by the matrix processing section 46. Note that $\alpha$, $\beta$ and $\gamma$ of equation (6) are matrix coefficients, and Rout, Gout and Bout show outputs of the R image, the G image and the B image after the matrix processing.

In response to the color tone which is desired as narrow band observation, $\alpha$, $\beta$ and $\gamma$ can be changed. The coefficients $\alpha$, $\beta$ and $\gamma$ are found in a range of, for example, 0.7 to 1.5 so as not to be excessively large or excessively small, and are selected from a plurality of candidates. If the coefficients exceed the range, noise increases, and saturation easily occurs. Under the conditions, only $\alpha$ is determined in a range of 0.35 to 0.75 with consideration given to (1+a) of the above described equation (5). With the consideration, noise increase and saturation increase do not occur as a matter of course, but expansion of a bit width is necessary so that a dynamic range is not lost.

$$\begin{pmatrix} Rout \\ Gout \\ Bout \end{pmatrix} = \begin{pmatrix} 0 & \alpha & 0 \\ 0 & 0 & \beta \\ 0 & 0 & \gamma \end{pmatrix} \begin{pmatrix} Rt \\ Gt \\ Bt \end{pmatrix} \quad \cdots (6)$$

In the above described equation (6), Rt is multiplied by 0. Accordingly, in the above described equation (5), Rt = B2 is shown, but Rt = 0 may be adopted.

A D/A convertor 47 converts the output of the matrix processing section 46 into an analog signal, and outputs the analog signal to the monitor 5. Namely, Rout, Gout and Bout of equation (6) are given to the monitor 5 as the RGB inputs. The monitor 5 displays the picked-up image in color in response to the RGB inputs which are inputted. In this manner, narrow band observation is enabled on the display screen of the monitor 5.

Next, an operation of the embodiment which is configured as above will be described.

A surgeon connects the connector 27 of the endoscope 2 to the light source apparatus 3 and the image processing apparatus 4 as shown in Fig. 1 on the occasion of use of the endoscope apparatus 1. Thereby, the connecting state shown in Fig. 1 is obtained. The surgeon operates the power supply switch not illustrated to bring each of the light source apparatus 3, the image processing apparatus 4 and the monitor 5 into an operating state, and performs operation for narrow band observation.

The light that is irradiated from the light source 31 is converted into the G illuminating light, the B1 illuminating light and the B2 illuminating light of a narrow band by the narrow band filter 32 and the rotation filter 33, and the brightness thereof is adjusted by the diaphragm 34, after which, the G illuminating light, the B1 illuminating light and the B2 illuminating light are supplied to the endoscope 2. The respective illuminating lights are irradiated to a subject side from the illumination lens 23 sequentially and substantially continuously at periods of 1/20 seconds, for example, via the light guide fiber 26.

In each timing at which the same site of the subject is irradiated with the G illuminating light, the B1 illuminating light and the B2 illuminating light, the CCD 25 picks up an optical image by the return light from the site. By photoelectric conversion of the CCD 25, the G signal, the B1 signal and the B2 signal corresponding to the respective return lights of the G illuminating light, the B1 illuminating light and the B2 illuminating light are obtained. The video signal including the G signal, the B1 signal and the B2 signal are given to the image processing apparatus 4 from the endoscope 2.

Note that the B1 signal and the B2 signal are the signals that are obtained by the image being picked up with the same exposure amount with use of the illuminating lights of the same wavelength band, and are obtained under the substantially same conditions except that a short timing deviation is present within one frame.

After predetermined analog processing is applied to the video signal inputted in the image processing apparatus 4 by the analog processing section 41, the video signal is converted into a digital signal by the A/D convertor 42. The digital video signal from the A/D convertor 42 is separated into the G signal, the B1 signal and the B2 signal in the digital processing section 43, and the separated signals are stored in the synchronization memory 40a.

The brightness calculation processing section 44 is given the G signal, the B1 signal and the B2 signal which are read from the synchronization memory 40a, and calculates the luminance Y1 by the master illumination and the luminance Y2 by the slave illumination by using the matrix processing of the matrix processing section 52. Next, the light adjustment control section 49 finds the difference value ΔY1 between the target luminance Ys and the luminance Y1, and finds the synthesis ratio a. As described above, the luminances Y1 and Y2 are calculated with use of the matrix processing, and show the brightnesses similar to the brightnesses in the case of being displayed on the monitor 5.

The synthesis ratio a is supplied to the synthesis processing section 45, and the synthesis processing section 45 synthesizes the picked-up image by the slave illumination by the ratio based on the synthesis ratio a, and the picked-up image by the master illumination. For example, the synthesis processing section 45 obtains the synthesized signal by using the above described equation (5). When the synthesis ratio a is zero, that is, the luminance Y1 is the target luminance Ys or more in equation (5), synthesis of the picked-up image by the slave illumination is not performed. Accordingly, in this case, blurring does not occur to the synthesized image based on the synthesized signal, and image quality degradation does not occur. Further, when the picked-up image by the slave illumination is synthesized, synthesis is performed at the ratio corresponding to the synthesis ratio a, and minimum required synthesis is performed to obtain a necessary brightness, whereby degradation of the image quality of the synthesized image can be suppressed.

The synthesized signal from the synthesis processing section 45 is given to the matrix processing section 46 and is subjected to matrix processing, and the signals of the R image, the G image and the B image in a display system are obtained. The outputs of the matrix processing section 46 are returned to analog signals by the D/A convertor 47, and thereafter, are supplied to the monitor 5. In this manner, the narrow band observation image with a sufficient brightness and image quality degradation being suppressed is displayed on the display screen of the monitor 5.

As above, according to the present embodiment, the same site to be the object to be observed is irradiated with the illuminating lights of the same narrow band a plurality of times in the predetermined time period such as one frame period and synthesis is performed, whereby the brightness of the image in the narrow band observation is enhanced. In this case, the brightness of the image is found with use of the matrix processing, whereby detection of the brightness corresponding to the brightness of the image which is actually displayed in the monitor is enabled, and observation with the brightness desired by a surgeon is enabled. Further, synthesis of the picked-up image by the same narrow band illuminating light is controlled with use of the synthesis ratio corresponding to the brightness of the detected image, and the minimum synthesis processing for obtaining the set brightness is performed, whereby degradation of the image quality can be suppressed.

Note that in the above described embodiment, the example of performing synthesis processing by the synthesis processing section 45, and performing matrix processing by the matrix processing section 46 is described, but the synthesis processing and the matrix processing can be performed in combination by matrix processing of one time. Further, synthesis processing may be performed after the matrix processing is performed.

(Second Embodiment)

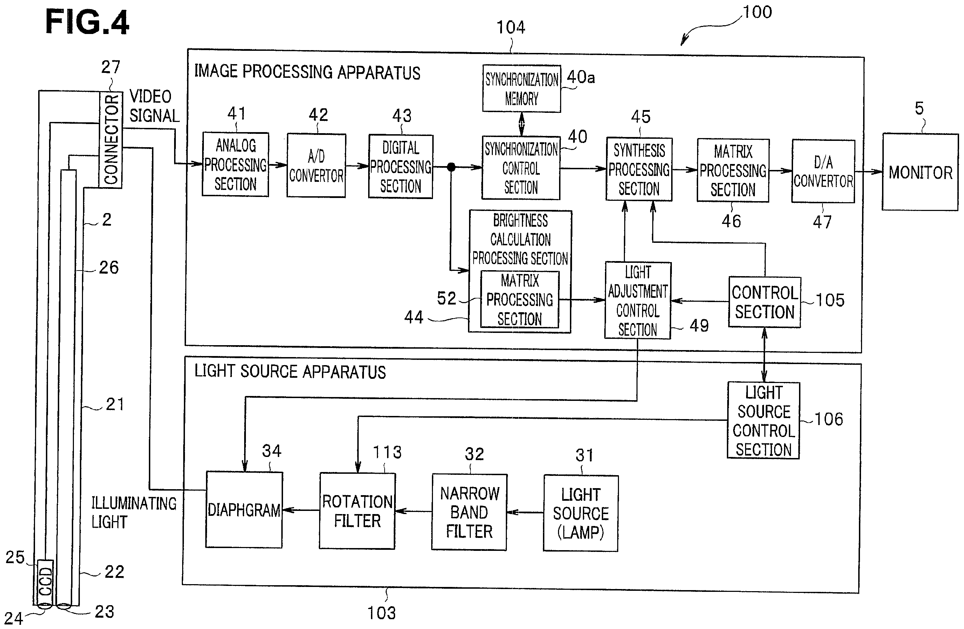

Fig. 4 is a block diagram showing a second embodiment of the present invention. In Fig. 4, the same components as in Fig. 1 are assigned with the same reference signs and the description will be omitted.

In the first embodiment, the example of adopting the rotation filter 33 for narrow band observation is described, whereas the present invention can be applied not only to narrow band observation but also to special light observation such as fluorescence observation. The present embodiment controls the synthesis ratio a in response to various observation modes, configurations of the light source apparatuses for realizing the observation modes, and the like. In order to respond to the configuration of the light source apparatus, determination by communication with the light source apparatus is conceivable.

An endoscope apparatus 100 according to the present embodiment differs from the first embodiment in the point that the endoscope apparatus 100 adopts an image processing apparatus 104 including a control section 105 in place of the image processing apparatus 4 and adopts a light source apparatus 103 including a light source control section 106 and a rotation filter 113, in place of the light source apparatus 3.

As the rotation filter 113, not only the rotation filter 33 of the first embodiment, but also a rotation filter for special observation such as fluorescence observation can be adopted. For example, as the rotation filter 113, a rotation filter provided with one or two filters for excitation light can be adopted, and a rotation filter provided with one or two filters for narrow band observation can be adopted.

The light source control section 106 retains various kinds of information relating to the light source apparatus 103, for example, information relating to a configuration of the rotation filter 113, and transmits and receives the retained information to and from the control section 105. Further, the light source control section 106 is controlled by the control section 105, and performs lighting control of the light source 31 and rotation control of the rotation filter 113. For example, the light source control section 106 performs control similar to the first embodiment when the rotation filter provided with two of filters for narrow band observation or filters for excitation light is adopted as the rotation filter 113, and controls the light source 31 and the rotation filter 113 so that the transmitted light of the filter is caused to exit at timing at which the image pickup signals of two channels are taken in when the rotation filter which is provided with only one of the filter for narrow band observation or the filter for excitation light is adopted as the rotation filter 113.

The control section 105 controls the synthesis processing section 45 and the light adjustment control section 49 based on various kinds of information relating to the light source apparatus 103 and the information relating to the observation mode designated by the operation of the operator. Further, the control section 105 controls the light source control section 106 in response to the observation mode.

Next, an operation of the embodiment configured as above will be described.

For example, when the rotation filter 33 of the first embodiment is adopted as the rotation filter 113, the control section 105 controls the respective sections so that the action similar to the first embodiment is performed. Note that when the synthesis ratio a satisfies $0 \le a < 1$, the control section 105 may control the light adjustment control section 49 so that the light adjustment control section 49 performs light adjustment control based on comparison of the luminance Y1 and the target luminance Ys.

Further, when the filter for irradiating an excitation light for fluorescence observation is adopted as the rotation filter 113, the control section 105 controls the respective sections based on the configuration of the rotation filter 113 based on the information from the light source control section 106. For example, when the rotation filter 113 has two filters for excitation light, the control section 105 sets the synthesis ratio a of the synthesis processing section 45 at one irrespective of the output of the brightness calculation processing section 44. Further, in this case, the control section 105 may control the light adjustment control section 49 so that the light adjustment control section 49 performs light adjustment control based on comparison of a luminance (Y1 + Y2) and the target luminance Ys.

Further, for example, when the filter which is provided with only one filter for excitation light is adopted as the rotation filter 113, the control section 105 controls the respective sections of the image processing apparatus 104 with consideration given to a sufficient brightness being not obtained, and stops the action of the fluorescence observation mode. In this case, the control section 105 may display on the monitor 5 a message indicating prohibition of the action in the designated observation mode.

Further, for example, when a filter which is provided with only one filter for narrow band observation is adopted as the rotation filter 113, the control section 105 sets the synthesis ratio a at zero. Similarly, when a filter which is provided with only one filter for special light observation is adopted as the rotation filter 113, the control section 105 sets the synthesis ratio a at zero.

Note that the control section 105 may set the synthesis ratio and the light adjustment control in response to the setting of the operator.

As above, in the present embodiment, the synthesis ratio is controlled in response to the kind of the light source apparatus, the observation mode and the like, and not only the effect similar to the first embodiment is obtained, but also optimal brightness control corresponding to the light source apparatus and the observation mode can be performed.

(Third Embodiment)

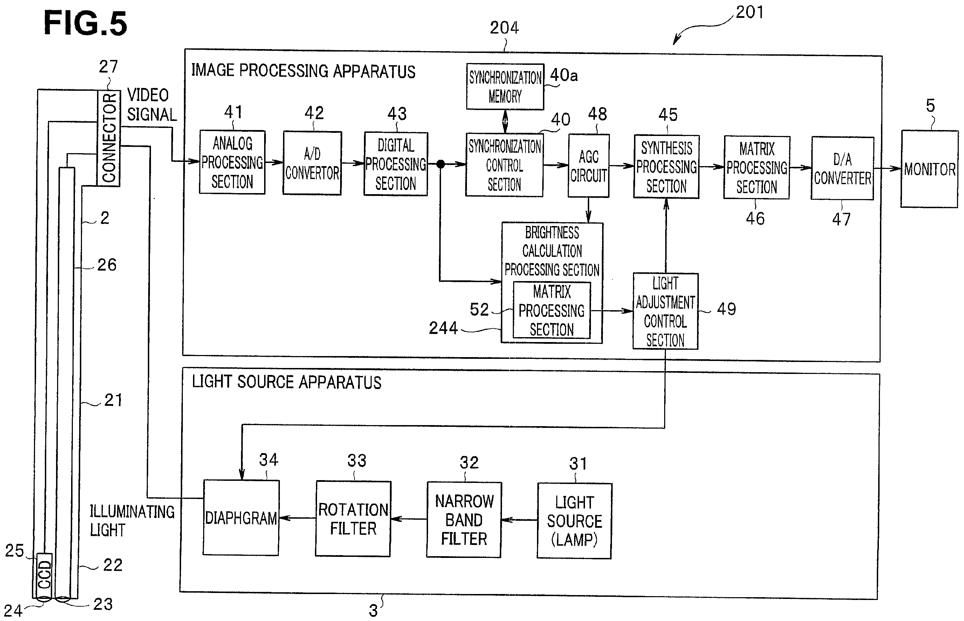

Fig. 5 is a block diagram showing an endoscope apparatus according to the third embodiment of the present invention. In Fig. 5, the same components as in Fig. 1 are assigned with the same reference signs and the description will be omitted.

The present embodiment differs from the first embodiment in the point that a brightness calculation processing section 244 is adopted in place of the brightness calculation processing section 44. The brightness calculation processing section 244 outputs a found brightness to the light adjustment control section 49 similarly to the brightness calculation processing section 44. In the present embodiment, the light adjustment control section 49 also finds the difference $(Ys - Y1) = \Delta Y1$ between the luminance Y1 obtained by the brightness calculation processing section 244 and the brightness (luminance) Ys as the target. In the case of $\Delta Y1 \leq 0$, the brightness as the target is obtained by only the master illumination, and therefore, the light adjustment control section 49 sets the synthesis ratio (coefficient) a of the picked-up image by the slave illumination at zero. Further, in the case of $\Delta Y1 > 0$, the light adjustment control section 49 determines the predetermined synthesis ratio a $(0 < a \leq 1)$. The light adjustment control section 49 outputs the synthesis ratio a to the synthesis processing section 45.

For example, when the synthesis ratio a satisfies $0 \leq a < 1$, the light adjustment control section 49 may perform light adjustment control based on comparison of the luminance Y1 and the target luminance Ys, and when the synthesis ratio a is one, the light adjustment control section 49 may perform light adjustment control based on comparison of the luminance Y1 + Y2 and the target luminance Ys.

Also in the present embodiment, the synthesis processing section 45 synthesizes the picked-up image by the master illumination and the picked-up image by the slave illumination based on the synthesis ratio a, and outputs the synthesized picked-up image (synthesized signal). Namely, the synthesis processing section 45 synthesizes the picked-up image by the slave illumination, that is, the signal obtained by the G signal and the B2 signal and the synthesis ratio a, and the signal based on the picked-up image by the master illumination. Note that when the synthesis ratio a is zero, the synthesis processing section 45 outputs the picked-up image by the master illumination, that is, the signal using only the G signal and the B1 signal to the matrix processing section 46 from the digital processing section 43.

The endoscope apparatus 1 in the present embodiment has a first mode in which the synthesis ratio a is, for example, zero, a second mode in which the synthesis ratio a is, for example, one, and a third mode in which the picked-up image by the slave illumination is multiplied by the synthesis ratio a $(0 < a < 1)$, and thereafter, the picked-up image by the slave illumination and the picked-up image by the master illumination are synthesized. Note that though the synthesis ratio a is described as zero or one in the first and the second modes, the synthesis ratio a may be set at values other than zero or one in the first and the second modes. Further, an example of having the three modes from the first to the third modes is described, but three kinds or more fixed synthesis ratios may be set as the synthesis ratio a, and the endoscope apparatus may be acted in the four or more modes.

Incidentally, the synthesis ratio a in the first mode is zero whereas the synthesis ratio a in the second mode is one, and control of the diaphragm closing amount by the light adjustment control section 49 is considered to be significantly different between the first mode and the second mode. Namely, in this case, the exit light quantities significantly differ, and the output levels of the CCD 25 significantly differ, between the first mode and the second mode. Therefore, when the brightness detection processing of the picked-up images based on the respective illuminating lights is the same between the first mode and the second mode, in the brightness calculation processing section 244, the detection result which does not correspond to the actual brightness is considered to be obtained.

Further, the light adjustment control section 49 performs light adjustment control based on the brightness of the picked-up image, whereas the brightness of an observed image that is displayed on the monitor 5 is based on the synthesized signal. Therefore, when it is determined that halation does not occur in brightness detection for light adjustment control, halation is likely to occur to the observed image on the monitor 5. Further, there is the possibility that a similar problem occurs depending on a gain amount of an AGC circuit 48 that acts independently of brightness calculation of the brightness calculation processing section 244.

Accordingly, in the present embodiment, threshold values for use in the brightness detection processing of the picked-up images based on the respective illuminating lights are changed between the first mode and the second mode so that brightness detection can be reliably performed even when the output levels of the CCD 25 significantly differ.

Further, in the present embodiment, the threshold values for use in the brightness detection processing of the picked-up images based on the respective illuminating lights are changed in response to the values of the synthesis ratio a and the gain of the AGC circuit 48 so that brightness detection corresponding to display can be reliably performed, whether the synthesis ratio a is large or small, or whether the gain of the AGC circuit 48 is large or small.

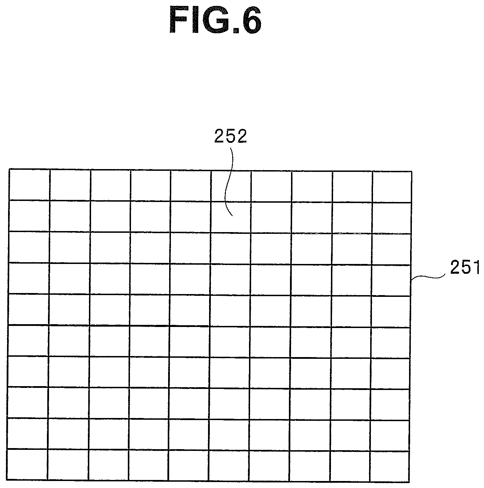

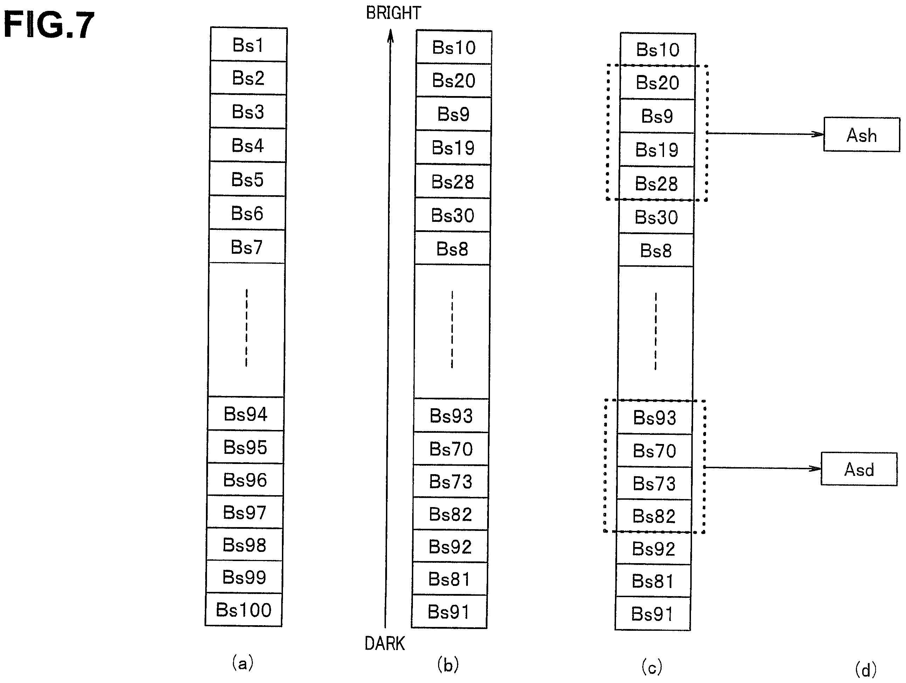

Fig. 6 and Fig. 7 are explanatory diagrams for explaining the brightness detection processing of the picked-up images based on the respective illuminating lights in the brightness calculation processing section 244 in Fig. 5. The brightness calculation processing section 244, for example, finds the brightness of the screen by dividing one screen into blocks with predetermined numbers of pixels. Note that the brightness calculation processing section 244 finds the brightness of the screen for each of the image pickup signals based on the respective illuminating lights.

Fig. 6 shows that an effective pixel region 251 is divided into 10 × 10 of blocks 252 by the brightness calculation processing section 244. The respective blocks 252 include predetermined numbers of pixels in a horizontal and a vertical directions. The brightness calculation processing section 244 finds the brightness at each of the blocks. For example, the brightness calculation processing section 244 sets an average of the pixel values of the pixels included in the respective blocks as the brightnesses of the respective blocks (block brightnesses). Now, the 100 blocks included in one screen are set as blocks B1 to B100, and the brightnesses of the respective blocks B1 to B100 are set as Bs1 to Bs100.

Fig. 7(a) shows the brightnesses Bs1 to Bs100 of the respective blocks B1 to B100 which are detected by the brightness calculation processing section 244 are arranged in sequence of the blocks. The brightness calculation processing section 244 arranges the brightnesses Bs1 to Bs100 in sequence of the magnitude of the values as shown in Fig. 7(b). In an example of Fig. 7(b), the brightness Bs10 of the block B10 is the highest, subsequently, the brightnesses become lower in sequence of Bs20, Bs9, ..., and the block B91 is the block with the lowest brightness Bs91.

Next, the brightness calculation processing section 244 selects regions that are enclosed by thick broken lines in Fig. 7(c), that is, the second to the fifth brightnesses in sequence of the brightness, and the 94th to the 97th brightnesses in sequence of the brightness. Subsequently, the brightness calculation processing section 244 sets an average value of the second to the fifth brightnesses in sequence of the brightness as a representative value (hereinafter, called a high luminance detection value) Ash of a bright region in the screen, and sets an average value of the 94th to the 97th brightnesses in sequence of the brightness as a representative value (hereinafter, called a low luminance detection value) Asd of a dark region in the screen. Note that which brightnesses in a high order and a low order are selected in order to find the high luminance detection value and the low luminance detection value can be properly set.

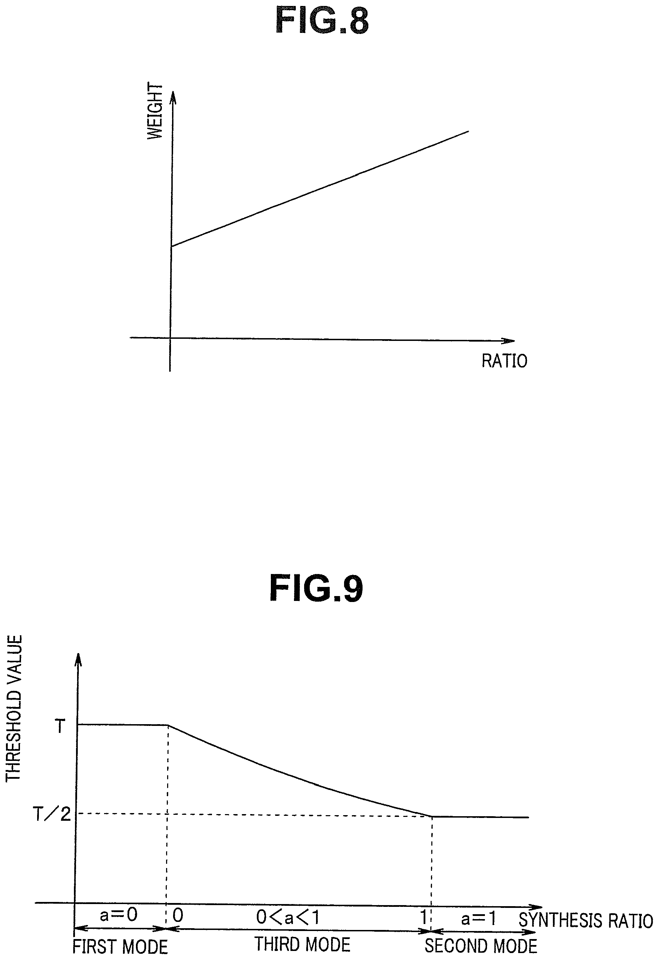

The brightness calculation processing section 244 makes addition by assigning a weight that changes in accordance with the mode to at least one of the high luminance detection value and the low luminance detection value, and thereby, finds the brightness of the screen. Fig. 8 and Fig. 9 are graphs for explaining the weight which changes in accordance with the modes. Fig. 8 shows the change of the weight, with the ratio plotted on the axis of abscissa, and the weight plotted on the axis of ordinates. Further, Fig. 9 shows the change of the threshold value to the mode and the synthesis ratio with the mode (synthesis ratio) plotted on the axis of abscissa, and the threshold value plotted on the axis of ordinates. Note that Fig. 8 and Fig. 9 are for explaining the weight which is given to a high luminance detection value.

The pixel values of all the pixels of the respective screens are also given to the brightness calculation processing section 244. The brightness calculation processing section 244 finds a ratio of the pixels the pixel values of which are a threshold value or more among all the pixels. For example, a value for use in halation determination of whether or not halation occurs to the pixels is set as the threshold value. In this case, the brightness calculation processing section 244 finds the ratio of the pixels in which halation occurs among all the pixels. In the example of Fig. 8, the brightness calculation processing section 244 makes the weight larger as the ratio of the pixels the pixel values of which are the threshold value or more (hereinafter, called high luminance pixels) is higher, as in the case in which halation occurs.

The brightness calculation processing section 244 sets a value that is obtained by multiplying the high luminance detection value by the weight based on Fig. 8, and thereafter, adding the result to the low luminance detection value, as the brightness of the screen. Accordingly, as the ratio of the high luminance pixels the pixel values of which are the threshold value or more is higher as in the case in which halation occurs, the high luminance detection value is multiplied by a larger weight, and the detection result indicating a brighter screen is obtained.

In the present embodiment, the threshold value for finding the ratio of the high luminance pixels is changed in response to the mode, the synthesis ratio and the gain of the AGC circuit 48. Note that Fig. 8 shows only the change of the threshold value with respect to the mode and the synthesis ratio. If, for example, the threshold value in the time of the first mode in which the synthesis ratio a is zero is set as T in Fig. 8, a threshold value in a time of the second mode in which the synthesis ratio a is one is set as T/2. Further, a threshold value T' in a time of the third mode in which the synthesis ratio a is $0 < a < 1$ is set as $T' = T/(1 + a)$.

In the first mode, for example, only the picked-up image by the master illumination is obtained. Therefore, the light adjustment control section 49 reduces the diaphragm closing amount and increases the exit light quantity of the light source apparatus 3. Accordingly, in this case, the output of the CCD 25 reaches a high level. On the other hand, in the second mode, the picked-up images by the master illumination and the slave illumination are synthesized. Consequently, a relatively bright (synthesized) picked-up image is obtained, and therefore, the light adjustment control section 49 increases the diaphragm closing amount, and decreases the exit light quantity of the light source apparatus 3. Accordingly, in this case, the output of the CCD 25 is at a relatively low level.

Accordingly, at the time of the second mode, the threshold value for finding the ratio of the high luminance pixels is set to be lower than at the time of the first mode, with consideration given to the picked-up images by the B1 and B2 illuminating lights being synthesized. Thereby, the brightness calculating processing section 244 properly determines the ratio of the high luminance pixels and can perform accurate brightness detection, irrespective of the mode.

Further, at the time of the third mode, as the synthesis ratio a becomes larger, the threshold value T' is made smaller. Thereby, at the time of the third mode, the brightness calculation processing section 244 also properly determines the ratio of the high luminance pixels, and can perform accurate brightness detection irrespective of the synthesis ratio a.

Further, at the time of the third mode, control may be performed with the gain of the AGC circuit 48 set as g, and the threshold value T' set as T' = T/g. In this case, as the gain g of the AGC circuit 48 becomes larger, the threshold value T' becomes smaller. Thereby, at the time of the third mode, the brightness calculation processing section 244 also properly determines the ratio of the high luminance pixels, and can perform accurate brightness detection, irrespective of the gain g of the AGC circuit 48.

The brightness calculation processing section 244 finds the brightness of the picked-up images based on the respective illuminating lights for each of the respective screens in this manner, and thereafter, calculates the luminances Y1 and Y2 by the matrix calculation of equation (1) and the calculations of the equations (2) and (3) which are described above.

Note that the example of assigning the weight to the high luminance detection value is described, but the weight by which a low luminance detection value is multiplied may be found. In this case, for example, the brightness calculation processing section 244 finds the ratio of the pixels (hereinafter, called low luminance pixels) the pixel values of which are a threshold value or less among all the pixels. Subsequently, the brightness calculation processing section 244 can change the threshold value for obtaining the ratio of the low luminance pixels in response to the mode.

Next, an operation of the embodiment which is configured as above will be described.

On the occasion of use of the endoscope apparatus 1, a surgeon connects the connector 27 of the endoscope 2 to the light source apparatus 3 and the image processing apparatus 204 as shown in Fig. 5. Thereby, the connection state shown in Fig. 5 is obtained. The surgeon operates a power supply switch not illustrated to bring each of the light source apparatus 3, the image processing apparatus 204 and the monitor 5 into an acting state, and performs the operation for narrow band observation.

The light that is irradiated from the light source 31 is converted into the G illuminating light, the B1 illuminating light and the B2 illuminating light of the narrow band by the narrow band filter 32 and the rotation filter 33, and after the brightnesses of the illuminating lights are adjusted by the diaphragm 34, the illuminating lights are supplied to the endoscope 2. The respective illuminating lights are irradiated to the subject side from the illumination lens 23 sequentially and substantially continuously in a period of 1/20 seconds, for example, via the light guide fiber 26.

In each timing at which the same site of the subject is irradiated with the G illuminating light, the B1 illuminating light and the B2 illuminating light, the CCD 25 picks up images of optical images by the return lights from the site. By photoelectric conversion of the CCD 25, the G signal, the B1 signal and the B2 signal corresponding to the respective return lights of the G illuminating light, the B1 illuminating light and the B2 illuminating light are obtained. The video signal including the G signal, the B1 signal and the B2 signal are given to the image processing apparatus 204 from the endoscope 2.

Note that the B1 signal and the B2 signal are the signals that are obtained by images being picked up with the same exposure amount with use of the illuminating lights of the same wavelength band, and are obtained under substantially the same conditions except that a short timing shift is present in one frame.

After predetermined analog processing is applied to the video signal which is inputted in the image processing apparatus 204 by the analog processing section 41, the video signal is converted into a digital signal by the A/D convertor 42. The digital video signal from the A/D convertor 42 is separated into the G signal, the B1 signal and the B2 signal in the digital processing section 43 to be stored in the synchronization memory 40a.

The brightness calculation processing section 244 is given the G signal, the B1 signal and the B2 signal which are read from the synchronization memory 40a, and finds the brightnesses of the picked-up images based on the respective illuminating lights at each of the respective screens. The brightness calculation processing section 244 finds the brightness of each block with respect to the respective screens of the picked-up images based on the respective illuminating lights. The brightness calculation processing section 244 finds the average of the block brightnesses with high-order brightnesses, and sets the average as the high luminance detection value, and finds the average of the block brightnesses with the low-order brightnesses, and sets the average as the low luminance detection value. The brightness calculation processing section 244 multiplies at least one of the high luminance detection value and the low luminance detection value by the weight which is found in response to the mode, the synthesis ratio a or the gain of the AGC circuit 48 and makes addition, and thereby finds the brightness of each of the respective screens with respect to the picked-up images based on the respective illuminating lights.

Now, the mode is assumed to be the first mode using only the picked-up image by the master illumination. In this case, the brightness calculation processing section 244 uses a relatively high value as the threshold value for finding the ratio of the high luminance pixels, for example. Thereby, the high luminance pixels can be detected with high precision. The brightness calculation processing section 244 makes the weight by which the high luminance detection value is multiplied larger, as the ratio of the high luminance pixels is higher. The brightness calculation processing section 244 multiplies the high luminance detection value by the weight that is found, and thereafter adds the result to the low luminance detection value to find the brightness of the screen.

In the case of the second mode which synthesizes the picked-up images by the master illumination and the slave illumination with the synthesis ratio a = 1, the brightness calculation processing section 244 uses a relatively low value as the threshold value for finding the ratio of the high luminance pixels, for example. Thereby, high luminance pixels can be detected with high precision. As the ratio of the high luminance pixels is higher, the brightness calculation processing section 244 makes the weight by which the high luminance detection value is multiplied larger. The brightness calculation processing section 244 multiplies the high luminance detection value by the weight which is found, and thereafter, adds the result to the low luminance detection value to find the brightness of the screen.

Further, in the case of the third mode in which the synthesis ratio a is in the range of 0 < a < 1, the threshold value is changed in response to the synthesis ratio a. Thereby, the high luminance pixels can be detected with high precision. As the ratio of the high luminance pixels is higher, the brightness calculation processing section 244 makes the weight by which the high luminance detection value is multiplied larger. The brightness calculation processing section 244 multiplies the high luminance detection value by the weight which is found, and thereafter, adds the result to the low luminance detection value to find the brightness of the screen.

Further, the brightness calculation processing section 244 may change the threshold value based on the gain of the AGC circuit 48. In this case, the high luminance pixels can be also detected with high precision. As the ratio of the high luminance pixels is higher, the brightness calculation processing section 244 makes the weight by which the high luminance detection value is multiplied larger. The brightness calculation processing section 244 multiplies the high luminance detection value by the weight which is found, and thereafter, adds the result to the low luminance detection value to find the brightness of the screen.

As above, the threshold value for finding the ratio of the high luminance pixels changes in response to the mode, the synthesis ratio a or the gain of the AGC circuit 48, and therefore, the brightness of the screen can be found with high precision irrespective of the mode.

The brightness calculation processing section 244 calculates the luminance Y1 by the master illumination and the luminance Y2 by the slave illumination by, for example, the matrix calculation by the above described equation (1) and the above described equations (2) and (3), by using the brightness which is found for each of the respective screens with respect to the picked-up images based on the respective illuminating lights. As described above, the luminances Y1 and Y2 are calculated with use of the matrix processing, and show the brightness similar to the brightness in the case of being displayed on the monitor 5. Next, the light adjustment control section 49 finds the difference value ΔY1 of the target luminance Ys and the luminance Y1, and determines the synthesis ratio a.

The synthesis ratio a is supplied to the synthesis processing section 45, and the synthesis processing section 45 synthesizes the picked-up image by the master illumination, and the picked-up image by the slave illumination by the ratio based on the synthesis ratio a. For example, the synthesis processing section 45 obtains the synthesized signal by using the above described equation (5). When the synthesis ratio a is zero, that is, the luminance Y1 is the target luminance Ys or more in equation (5), synthesis of the picked-up image by the slave illumination is not performed. Accordingly, in this case, blurring does not occur to the synthesized picked-up image based on the synthesized signal, and image quality degradation does not occur.

The synthesized signal from the synthesis processing section 45 is given to the matrix processing section 46 and is subjected to the matrix processing, and the signals of the R image, the G image and the B image in the display system are obtained. The output of the matrix processing section 46 is returned to an analog signal by the D/A convertor 47, and thereafter, is supplied to the monitor 5. In this manner, on the display screen of the monitor 5, the narrow band observation image with a sufficient brightness and image quality degradation being suppressed is displayed.

As above, according to the present embodiment, the same site to be an object to be observed is irradiated with the illuminating lights of the same narrow band a plurality of times in the predetermined time period such as one frame period, and synthesis is performed, whereby the brightness of the image in narrow band observation is enhanced. In this case, the brightness of the screen is found with use of the threshold value corresponding to the mode, the synthesis ratio or the AGC gain, and the brightness of the screen can be found with high precision. Further, the brightness of the image by the picked-up images based on the respective illuminating lights can be found with use of the matrix processing, whereby the brightness detection corresponding to the brightness of the image which is actually displayed on the monitor is enabled, and observation with the brightness desired by a surgeon is enabled. Further, when the brightness of the image is sufficiently bright, synthesis of the picked-up images by the illuminating lights of the same narrow band is not performed, and therefore, degradation of the image quality can be suppressed.

Note that in the above described embodiment, the example is described, in which synthesis processing is performed by the synthesis processing section 45, and the matrix processing is performed by the matrix processing section 46, but the synthesis processing and the matrix processing are combined and can be performed by the matrix processing of one time. Further, after the matrix processing is performed, synthesis processing may be performed.

Further, in the above described embodiment, the example of changing the threshold value for finding the ratio of the high luminance pixels in accordance with the mode and the like is described, but various threshold values for finding the brightness of the screen may be changed in accordance with the mode, the synthesis ratio or the AGC gain.

For example, in the above described embodiment, the example is described, in which a weight is calculated with use of the threshold value corresponding to the mode or the like when the screen is divided into a plurality of blocks, the brightness is found for each of the respective blocks, and the weight is assigned to the representative value of a plurality of block brightnesses, but the threshold value for use in determination of the representative value of the block brightnesses can be also changed in response to the mode, the synthesis ratio or the AGC gain, as in the case of finding the representative value with respect to a plurality of block brightnesses from which the block brightnesses having the brightnesses not more than the threshold value corresponding to the mode or the like are eliminated, when the representative value of a plurality of block brightnesses is found.

Namely, the output level of the image pickup device changes in response to the mode or the like, and therefore, in the case of finding the brightness of the screen, when the threshold value relating to the output level of the image pickup device, that is, the threshold value for determining whether a luminance is a high luminance or a low luminance in a pixel unit or a block unit needs to be set, the threshold value can be changed in response to the mode, the synthesis ratio or the AGC gain.

Note that in the above described embodiment, the example of the narrow band observation is described, but the present invention is similarly applicable in fluorescence observation, and when a rotation filter having two filters for excitation light for performing fluorescence observation is used, the synthesis processing and the brightness calculation processing similar to the above described embodiment can be performed.

(Fourth Embodiment)

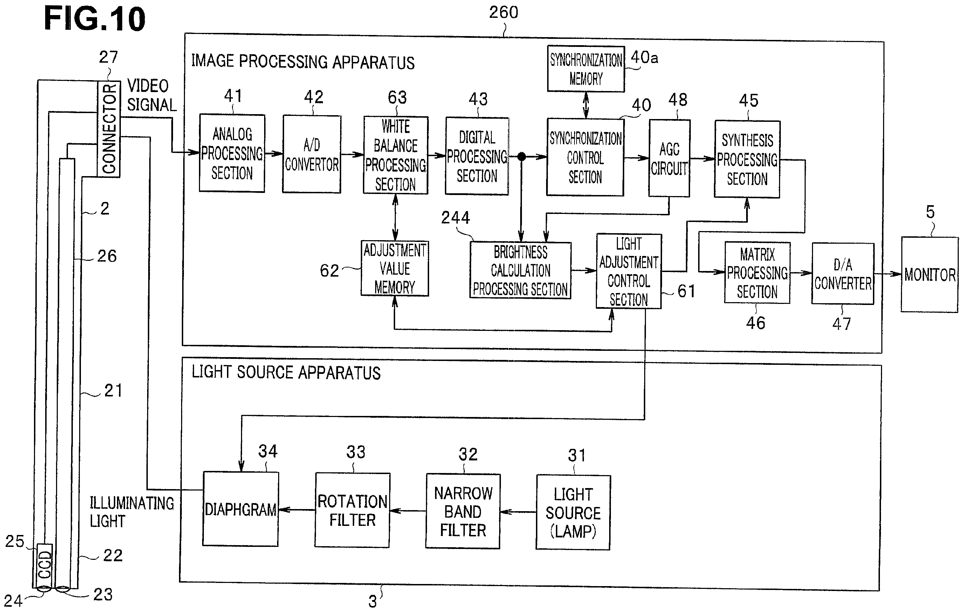

Fig. 10 is a block diagram showing a fourth embodiment of the present invention. In Fig. 10, the same components as in Fig. 5 are assigned with the same reference signs, and the description thereof will be omitted.

In an endoscope apparatus for performing narrow band observation, white balance adjustment is necessary so that a hue displayed on the monitor is in a desired state similarly to the endoscope apparatus which performs normal light observation. For example, in the aforementioned third embodiment, the diaphragm closing amount sometimes significantly changes in response to the mode or in response to the synthesis ratio a. In that case, the color of the exit light from the light source apparatus 3 sometimes changes in accordance with the change of the diaphragm closing amount. Accordingly, white balance adjustment corresponding to the mode and the synthesis ratio a is required.

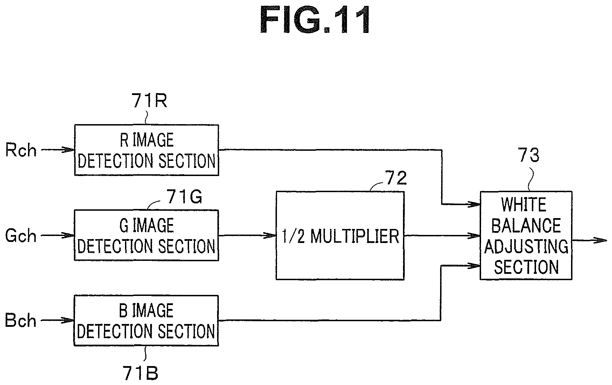

Fig. 11 is a block diagram showing an ordinary circuit that performs white balance adjustment. A signal Rch of the R image, a signal Gch of the G image and a signal Bch of the B image are respectively given to an R image detection section 71R, a G image detection section 71G and a B image detection section 71B and are detected. Note that for narrow band observation in the present embodiment, the B signal, the G signal and the B signal are used as the signals of the R, G and B images.

In the circuit of Fig. 11, with consideration given to the case in which the brightness of the G illuminating light is extremely higher than the B illuminating light, for example, the G signal is multiplied by a predetermined coefficient, for example, 1/2, in a multiplier 72. The B signal from the R image detection section 71R, the G signal from the multiplier 72 and the B signal from the B image detection section 71B are given to a white balance adjusting section 73, and are multiplied by a predetermined gain to be subjected to white balance adjustment.

Note that the white balance adjustment in narrow band observation is shown, and in fluorescence observation, similar color balance adjustment can be performed with use of a circuit similar to Fig. 11. For example, when a rotation filter having two filters for excitation light is adopted, the G light is irradiated at timing when the signal of the R image is obtained, a first excitation light is irradiated at timing when the signal of the G image is obtained, and a second excitation light is irradiated at timing when the signal of the B image is obtained. Color balance adjustment is performed with the detection result being reduced by half for the G signal based on the irradiation of the G light, and with use of the detection result as it is for the first and the second fluorescence signals based on the first and the second excitation lights.

However, when the method is adopted, which acquires the white balance adjustment value by only adjustment of the signal gains with respect to the image pickup signals based on the respective narrow band illuminating lights to be adapted to the circuit of Fig. 11, the light quantity of the G illuminating light is sometimes extremely large relatively to the B illuminating light which is caused to exit from the light source apparatus 3, depending on the characteristics of the narrow band filter 32. In this case, for example, the gains of the B1 and the B2 signals need to be made extremely large as compared with the gain of the G signal. By doing so, an effective level range as the B1 and the B2 signals becomes narrow, and the dynamic range becomes narrow.

Accordingly, in the present embodiment, on the occasion of acquisition of the white balance adjustment values, the exit light quantities of the respective bands are controlled in accordance with the mode, whereby the dynamic range is prevented from becoming narrow.

The present embodiment differs from the third embodiment in the point that an image processing apparatus 260 is adopted in place of the image processing apparatus 204. The image processing apparatus 260 differs from the image processing apparatus 204 of the third embodiment in the point that a light adjustment control section 61 is adopted in place of the light adjustment control section 49, and an adjustment value memory 62 and a white balance processing section 63 are added.

The light adjustment control section 61 has a function similar to that of the light adjustment control section 49 of the third embodiment, and has a function of controlling acquisition processing of the white balance adjustment value. The first mode and the second mode differ in the synthesis ratio a. For example, the synthesis ratio a in the first mode is set as zero, and the synthesis ratio a in the second mode is set as one. In this case, in the first mode, picked-up images by the G illuminating light and the B 1 illuminating light are obtained. When a light quantity of the G illuminating light tends to be larger than a light quantity of the B1 illuminating light by the characteristics or the like of the narrow band filter 32, the light adjustment control section 61 increases the diaphragm closing amount of the diaphragm 34 at timing when the light from the light source 31 passes through the filter 33G of the rotation filter 33, and reduces the diaphragm closing amount of the diaphragm 34 at timing when the light from the light source 31 passes through the filter 33B1 of the rotation filter 33, at a time of acquisition of the white balance adjustment value corresponding to the first mode.

Further, in the second mode, the picked-up images by the G illuminating light, the B 1 illuminating light and the B2 illuminating light are synthesized. Accordingly, it is conceivable that regarding the picked-up image based on the B illuminating light, a sufficient level can be also obtained in relation to the G picked-up image, and therefore, the light adjustment control section 61 makes the diaphragm closing amount of the diaphragm 34 the same, for example, at timing when the light from the light source 31 passes through the filter 33G of the rotation filter 33 and timing when the light from the light source 31 passes through the filters 33B1 and B2, at the time of acquisition of the white balance adjustment value corresponding to the second mode.

The white balance processing section 63 finds the respective white balance adjustment values for the first and the second modes based on the output of the A/D convertor 42, and causes the adjustment value memory 62 to store the respective white balance adjustment values, at the time of acquisition of the white balance adjustment values. The light adjustment control section 61 corrects the respective white balance adjustment values for the first and the second modes based on the diaphragm closing amounts at the time of acquisition of the white balance adjustment values corresponding to the first and the second modes and causes the adjustment value memory 62 to store the respective white balance adjustment values.

Note that control of the diaphragm closing amount at the time of acquisition of the white balance adjustment value can be determined based on the level of the picked-up image based on the G illuminating light and the level of the picked-up image based on the B illuminating light. The picked-up image based on the B illuminating light is obtained by the picked-up image based on the B 1 illuminating light and the picked-up image based on the B2 illuminating light being synthesized in accordance with the synthesis ratio a. Accordingly, the diaphragm closing amounts at the time of acquisition of the white balance adjustment values corresponding to the fist and the second modes can be set based on the synthesis ratios a at times of the first and the second modes.

Note that the light adjustment control section 61 adjusts the light quantity by controlling the diaphragm closing amount, but may adjust the exit light quantity of the light source 31.

In the embodiment configured as above, the white balance adjustment value is determined for each mode. At the time of acquisition of the white balance adjustment value corresponding to the first mode, the light adjustment control section 61 restricts the light quantities of the G illuminating light and the B illuminating light with the diaphragm closing amount based on the synthesis ratio a which is set for the first mode. The white balance processing section 63 calculates the white balance adjustment value and outputs the white balance adjustment value to the adjustment value memory 62. The light adjustment control section 61 reads the white balance adjustment value for the first mode stored in the adjustment value memory 62, corrects the white balance adjustment value in response to the diaphragm closing amount, and thereafter, causes the adjustment value memory 62 to store the white balance adjustment value.

Further, at the time of acquisition of the white balance adjustment value corresponding to the second mode, the light adjustment control section 61 restricts the light quantities of the G illuminating light and the B illuminating light with the diaphragm closing amount based on the synthesis ratio a which is set for the second mode. The white balance processing section 63 calculates the white balance adjustment value, and outputs the white balance adjustment value to the adjustment value memory 62. The light adjustment control section 61 reads the white balance adjustment value for the second mode which is stored in the adjustment value memory 62, corrects the white balance adjustment value in response to the diaphragm closing amount, and thereafter, causes the adjustment value memory 62 to store the white balance adjustment value.

At a time of actual use, the white balance processing section 63 reads the white balance adjustment value for the first or the second mode which is stored in the adjustment value memory 62 in response to the mode, and amplifies the image pickup signal. The other operations are similar to those of the third embodiment.

As above, in the present embodiment, white balance adjustment corresponding to the respective modes is performed, and at the time of acquisition of the white balance adjustment values, the light quantities of the illuminating lights of the respective bands are restricted in response to the synthesis ratios of the respective modes, whereby the white balance adjustment values of the respective modes can be acquired while the dynamic range is secured.

Note that in the above description, the first and the second modes are described, but it is obvious that a white balance adjustment value may be calculated by the aforementioned method for each synthesis ratio a of the third mode.

Further, the example of narrow band observation is described, but the present embodiment is obviously applicable similarly to color balance adjustment of fluorescence observation.

Incidentally, an endoscope is provided with a custom switch, and toggle of photometry and contrast, focus and the like are switchable with one switch. Namely, various operation switches are provided at a front panel, a keyboard and the like, and the custom switch which can be assigned with an optional function is present. Regarding a scope of an optical magnifying function, optical magnifying operation has been conventionally performed only by an exclusive operation lever, and the custom switch is assigned with the function, whereby the degree of freedom of the operation method can be enhanced. Regarding a scope of two-focal-points switching without use of an operation lever, two focal points switching can be assigned to a custom switch. Note that as for optical magnification and two-focal points switching, which state is brought about after operation can be displayed. For example, in order to show proximity (Near) and normality (Far) from two focal points, Near or Far can be displayed on the screen. At this time, in order to distinguish the display from display of a patient name and the like, white and black are inverted, or a large font is used, whereby recognizability can be enhanced.

Further, in each function, a plurality of modes and levels are switched with toggle. On such an occasion, at least one or more is selected from the plurality of modes and levels, whereby photometry and contrast can be easily switched with the custom switch. For example, photometry has three modes of average, peak and auto, and if the peak and the auto are set in a menu in advance, the peak and the auto can be always switched with the custom switch. Similarly, for contrast, normal and high are set from kinds that are normal, high , low and no correction, from a table of gamma correction, switching becomes easy. Further, in order to set contrast = normal as one candidate without fail, a choice may be made between only high and low in setting. At this time, switching operation between normal and special is made, and for the special, the contrast is set from the high and the low. The above may be carried out in various functional switches of photometry, contrast and the like that operate the functions, without being limited to the custom switch. On this occasion, the three modes of photometry are shown in the front panel, and switchable two modes sequentially glow, and the mode which is not switchable does not glow. Further, the way of glowing may be devised so that the switchable two modes can be distinguished from the mode which is not switchable so that the switchable two modes are recognizable. The mode which is selected is caused to glow strongly, the other mode that is selectable is caused to glow weakly, and the mode which is not switchable has the light extinguished.

Further, in response to the observation mode, the function to be realized and image processing may be changed in one custom switch or one functional switch. For example, concerning the functional switch for color, operation thereof changes a level of color enhancement in the normal observation mode, and changes the color tone mode in the NBI observation mode.

A scope ID stores the kind of the scope and the number of the NBI color tone mode. If the kind of the scope is a colonoscope, or the NBI color tone mode is three, a parameter corresponding to the NBI color tone mode = 3 is read from a matrix for NBI. Read of the parameter may be carried out from the kind of the scope for each communication method of the scope ID, and may be carried out from the NBI color tone mode. In the case of the former, the number of the NBI color tone mode can be automatically matched from the kind of the scope. Further, when no scope ID is present, NBI color tone mode = 2 is set, and thereby, the color tone which is applicable to both a colon and an esophagus can be realized.

Further, for the NBI color tone mode, by a variable switch, the NBI color tone mode can be changed from three to one, and from one to two. Thereby, the NBI color tone mode is set at one for an esophagus, and can be changed to two for a stomach. The NBI color tone mode can be adapted to the preferences of a user. Further, setting of the NBI color tone mode by the scope ID is not allowed to be made, and an NBI color tone mode which the user sets in advance may be used. For example, after the user sets the NBI color tone mode at two, the user turns off the system, and when the user turns on the system again, the user can call two which is used at the previous time. Whether the scope ID is used or not can be set in the menu.

A plurality of kinds of light sources can be combined with the processor. It is indicated that the observation modes which can be provided are different in accordance with the light sources. Depending on the kinds of the light sources, the communication sections with the processor differ, and therefore, the processor can have communication sections corresponding to the respective light sources. The processor can be provided with two communication sections with one connector. More specifically, it is preferable to use a plurality of pins in the connector properly, and it is more favorable to include pins that can be shared. The communication section of the processor can be changed in response to the light source, and the signal standards can be changed. For example, setting a synchronizing signal to be a composite synchronizing signal or a vertical synchronizing signal is cited.

The scope is electrically connected to the processor with a cable, and is electrically connected with the cable via the light source. On this occasion, the scope can be reattached or replaced while the power supply of the processor is on. In that case, when the state transitions from the state in which the scope is unconnected, to the state in which the scope is connected, it sometimes takes time after the rotation position of the color filter of the light source becomes unsuitable until the rotation position becomes suitable. In that case, the observation screen is disturbed. Therefore, when the scope is unconnected, the rotation position of the color filter immediately before the scope is unconnected is retained, and thereby proper observation is always enabled. The rotation position of the color filter shown here can be said to be exposure timing to the CCD in a wide sense. Namely, if an opening adjustor which is combined with the color filter makes the relative position in the rotational direction to the color filter proper, the exposure timing to the CCD becomes correct, and therefore, it is important to retain the positions by including them.

The switch provided on the front panel is pressed, whereby operation of the white balance is enabled. The white balance has to be executed for each of the normal observation mode and the NBI observation mode, and for example, the switch is kept pressed for approximately four seconds, whereby white balance can be acquired for each of the normal observation mode and the NBI observation mode in sequence. However, it is sometimes troublesome to keep pressing the switch, and therefore, the need to keep pressing the switch may be eliminated by setting. More specifically, setting is made in such a manner as "white balance switch holding = ON/OFF". If the setting is made OFF, white balance of the normal observation mode is started to be obtained after the switch is pressed for approximately one second, and when a notice indicating this is given, white balance is not intermitted even if the user stops holding the switch, and white balance of the NBI observation mode can be automatically obtained. When the setting is turned on, if the user stops holding the switch before white balance is totally completed, the white balance cannot be correctly completed, and the white balance value cannot be acquired.

Hold of the white balance switch by setting is described so far, but the white balance switch may be automatically switched in accordance with the kind of the endoscope to be used. The kind of the endoscope is detected by the scope ID, and when it is found out that the endoscope is a scope for surgery, action may be made to turn off holding of the white balance switch. This is because when the user who operates the switch differs from the user who holds the endoscope in the field of surgery, timing of operation of the switch is shifted, and holding sometimes becomes difficult. Further, when the endoscope is loaded with a CCD, and is also loaded with a resistance element or the like to show the kind of the CCD, the kind of the CCD is determined based on the resistance value, and thereafter, action may be made to turn off holding of the white balance switch in response to the kind of the CCD. Further, turning off holding of the white balance switch may be realized with use of setting and the scope ID. When the scope ID shows surgery, and further, in the setting, "white balance switch holding = OFF" is set, action may be made to turn off holding of the white balance switch. Note that in the case of a frame sequential endoscope, white balance has an operation of absorbing manufacturing variations of the color filter of the light source apparatus, and improving color reproducibility. In addition to this, on the occasion of the CCD of the endoscope being 4-channel reading, white balance has an operation of absorbing variations of the respective channels, and prevents variations among the channels. In the white balance in which both of the operations are incorporated, $3 + 4 - 1 = 6$ different white balance coefficients are cited in order to correspond to the three colors of RGB and the four channels of the CCD. This is because when the channels are set as 1 to 4, the coefficients of R1 to R4, G1 to G4 and B1 to B4 are needed.

As video outputs, various connectors such as SDI, DVI, and DV are included. For the video outputs, determined formats are present, and circuit blocks necessary for each of them are present therein. When an interlace signal is outputted to the SDI, and a progressive signal is outputted to the DVI, IP conversion (interlace-progressive conversion) becomes necessary as the circuit block for the DVI. While HDTV of interlace of horizontal 1920 dots and vertical 1080 lines is outputted to HD-SDI, the HDTV is subjected to IP conversion to be progressive of $1920 \times 1200$ of WUXGA, and is outputted to the DVI. When the HDTV of the interlace is only converted into the progressive, a difference is present between 1080 and 1200, and therefore, the difference is filled with a black blank. Further, in accordance with $1280 \times 1024$ of SXGA, the difference may be cut out. Further, on the occasion of IP conversion, a median filter is used, and thereby, image quality improvement can be made on the occasion of characters being displayed.

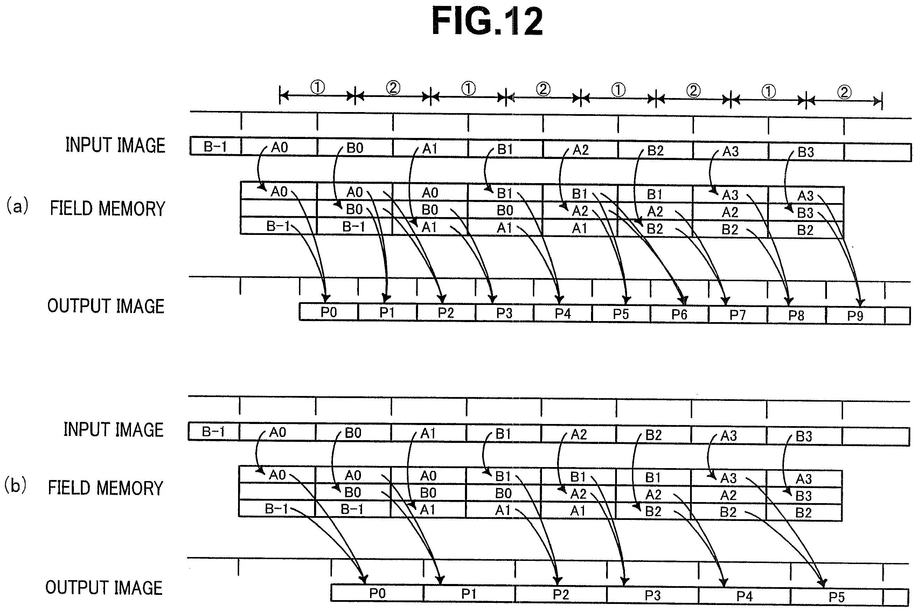

A state of conversion from interlace to progressive will be described with a timing chart of Fig. 12. Fig. 12(a) shows a case of an input frame rate to a field memory < an output frame rate, and Fig. 12(b) shows a case of the input frame rate to the field memory > the output frame rate. A circled figure 1 of Fig. 12 indicates a time period in which an image of an A field and an image of an immediately preceding B field are subjected to synthesis processing and thereafter outputted, and a circled figure 2 of Fig. 12 indicates a time period in which an image of the B field and the image of the immediately preceding A field are subjected to synthesis processing, and thereafter, outputted.

When a frequency of the output image of progressive is higher than interlace, the images shown in P5 and P6 are totally the same. Namely, in P2, P6 and the like, the same field is outputted twice, and thereby, the frequency shift is absorbed. When the frequency of the output image of progressive is lower than interlace, the interlace image which is not outputted is provided as between P1 and P2, and the frequency shift is absorbed.

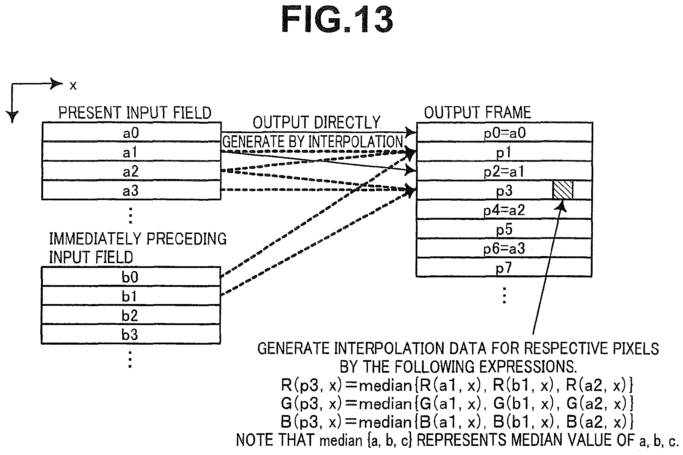

Next, an operation of the median filter will be described with an explanatory view of Fig. 13. The present input field is directly outputted by each line as the output frame. With respect to p1, p3, ... of the output frame, output of the median filter is used. For the output frames, the output in which the signal level is the second largest is sought in three of a0, b0 and a1, and is outputted as p1. If the image is a still image, b0 is the most suitable as p1, and if the image is a moving image, any one of a0 and a1 is suitable. This is the method that pays attention to them. The median filter is applied for each of the respective R, G and B, and is applied for each pixel. With the pixels in the oblique line portion of Fig. 13 taken as an example, in the case of the R image, the images with the signal level being the second largest is sought in three of (a1, x), (b1, x) and (a2, x) of the R image, and output thereof is obtained.

In NBI observation, ensurement of the light quantity is difficult. Accordingly, a plan to increase the light quantity by increasing the current which drives a lamp is considered. In normal light observation, a sufficient light quantity can be already ensured in many cases, and only in the case of a thin endoscope like a transnasal endoscope, the light quantity is desired to be increased. Accordingly, the scope ID is provided with a flag, and thereby, action is made to increase the light quantity or no action may be made. When setting is provided to increase the degree of freedom concerning such an action, and, for example, OFF is selected in such an item as "current restriction = ON/OFF", the aforementioned flag is invalid and the light quantity is increased, and if ON is selected, and if "1" is assigned to the aforementioned flag, the light quantity is increased, whereas if "0" is assigned, an action is made not to increase the light quantity.

While CCDs are essential to endoscopes, the kinds of endoscopes are various, and the circuits that drive the CCDs are included in the endoscopes or included in the video processors. When the CCD is driven by the circuit in an endoscope, the matters which are necessary for the circuit are given from the video processor. The matters are mainly a power supply, a clock and a ground. Further, there are various kinds of CCDs, and the voltages of the power supplies thereof and the frequencies of the clocks which are suitable for the CCDs are required. In order to transmit the kind of a CCD to the video processor, the endoscope is provided with a resistance value, where the voltage is recognized, and thereby, CCD discrimination is realized. Such an endoscope requires a certain amount of attention in connection with the video processor. For example, in regard with the length of contact pins that are in charge of connection, if the contact pins for the power supply, the clock and the ground, and the resistance section for CCD discrimination are made long, the contact pins for the other signals are made short, proper connection is also made while the video processor is energized. Without being limited thereto, the length of the contact pin for the signal which makes the kind of the endoscope recognizable may be made long. The effect thereof is that if the power supply, the clock and the ground are simultaneously detached when the endoscope is detached, the driver circuit for CCD, the circuits such as A/D and AFE which simultaneously require the power supply, the clock and the ground can be stabilized. Further, if the power supply and the resistance section are simultaneously connected when the endoscope is connected, a proper clock frequency is prepared, and can be sent to the endoscope.

Even while an endoscopic image is frozen and a still image is displayed, characters can be inputted into a comment column or the like. At this time, a patient ID cannot be inputted or changed. Further, whether an endoscopic image is frozen or not, GUI is displayed as a menu on the monitor screen in order to change the color of the endoscopic image, and fixed amounts of a red color and a blue color may be changed.

The present application is filed claiming the priority of Japanese Patent Application Nos. 2011-185127 filed in Japan on August 26, 2011, 2011-185128 filed in Japan on August 26, 2011.

**Claims**

1. An endoscope apparatus (1), comprising:

   illuminating means (3) configured to perform illumination by an illuminating light of a first band, and perform illuminations by an illuminating light of a second band for a first number of times that is two times or more, within a predetermined time period;
   image pickup means (25) configured to pick up an image of an object illuminated by the illuminating means (3), and output a first image pickup signal based on the illumination of the illuminating light of the first band, and image pickup signals based on the illuminations of the illuminating light of the second band; and
   brightness calculating means (44) configured to calculate a first brightness by color conversion matrix processing using the first image pickup signal and a second image pickup signal based on illumination by first predetermined times out of the first number of times, and calculate a second brightness by color conversion matrix processing using the first image pickup signal and a third image pickup signal based on illumination by times other than the first predetermined times out of the first number of times,
   **characterized by**

synthesizing means (45) configured to synthesize and output a picked-up image by multiplying the first and the third image pickup signals by a coefficient based on a ratio of a difference value of a target brightness and the first brightness, and the second brightness and thereafter synthesizing the result with the first and the second image pickup signals.

2.  The endoscope apparatus (1) according to claim 1,
    wherein the first number of times is two times,
    the brightness calculating means (44) are configured to calculate the first brightness by color conversion matrix processing using the first image pickup signal and the second image pickup signal based on illumination of a first time out of the first number of times, and calculate the second brightness by color conversion matrix processing using the first image pickup signal and the third image pickup signal based on illumination of a second time out of the first number of times.

3.  The endoscope apparatus (1) according to claim 2, further comprising:

    a matrix processing section (52) configured to perform color conversion processing corresponding to input of a display system that performs display based on the first to third image pickup signals,
    wherein synthesis by the synthesizing means is performed at a pre-stage or a post-stage of the matrix processing section, or performed simultaneously with the color conversion processing of the matrix processing section (52).

4.  The endoscope apparatus (1) according to claim 3,
    wherein the first band is a green band, and the second band is a blue band.

**Patentansprüche**

1.  Endoskopvorrichtung (1), umfassend:

    ein Beleuchtungsmittel (3), das so konfiguriert ist, dass es
    Beleuchtung durch ein Beleuchtungslicht eines ersten Bandes durchführt und Beleuchtungen durch ein Beleuchtungslicht eines zweiten Bandes durchführt, für eine erste Anzahl von Malen, die zweimal oder öfters ist, innerhalb eines vordefinierten Zeitraums;
    ein Bildaufnahmemittel (25), das so konfiguriert ist, dass es ein Bild eines mit dem Beleuchtungsmittel (3) beleuchteten Objekts aufnimmt und ein erstes Bildaufnahmesignal auf Basis der Beleuchtung des Beleuchtungslichts des ersten Bandes und Bildaufnahmesignale auf Basis der Beleuchtungen des Beleuchtungslichts des zweiten Bandes ausgibt; und
    ein Helligkeitsberechnungsmittel (44), das so konfiguriert ist, dass es eine erste Helligkeit durch eine Farbumwandlungsmatrixverarbeitung unter Verwendung des ersten Bildaufnahmesignals und eines zweiten Bildaufnahmesignals auf Basis einer Beleuchtung für erste vordefinierte Male der ersten Anzahl von Malen berechnet und eine zweite Helligkeit durch Farbumwandlungsmatrixverarbeitung unter Verwendung des ersten Bildaufnahmesignals und eines dritten Bildaufnahmesignals auf Basis einer Beleuchtung für Male anders als die ersten vordefinierten Male der ersten Anzahl von Malen berechnet,
    **gekennzeichnet durch**
    ein Synthetisierungsmittel (45), das so konfiguriert ist, dass es ein aufgenommenes Bild durch Multiplizieren des ersten und des dritten Bildaufnahmesignals mit einem Koeffizienten auf Basis eines Verhältnisses eines Differenzwerts einer Zielhelligkeit und der ersten Helligkeit, und der zweiten Helligkeit synthetisiert und ausgibt und danach das Ergebnis mit dem ersten und dem zweiten Bildaufnahmesignal synthetisiert.

2.  Endoskopvorrichtung (1) nach Anspruch 1,
    wobei die erste Anzahl von Malen zweimal ist,
    das Helligkeitsberechnungsmittel (44) so konfiguriert ist, dass es die erste Helligkeit durch eine Farbumwandlungsmatrixverarbeitung unter Verwendung des ersten Bildaufnahmesignals und des zweiten Bildaufnahmesignals auf Basis einer Beleuchtung für ein erstes Mal der ersten Anzahl von Malen berechnet und die zweite Helligkeit durch Farbumwandlungsmatrixverarbeitung unter Verwendung des ersten Bildaufnahmesignals und des dritten Bildaufnahmesignals auf Basis einer Beleuchtung für ein zweites Mal der ersten Anzahl von Malen berechnet.

3.  Endoskopvorrichtung (1) nach Anspruch 2, ferner umfassend:

einen Matrixverarbeitungsabschnitt (52), der so konfiguriert ist, dass er eine Farbumwandlungsverarbeitung entsprechend einer Eingabe eines Anzeigesystems durchführt, das eine Anzeige auf Basis des ersten bis dritten Bildaufnahmesignals durchführt,

wobei eine Synthese durch das Synthetisierungsmittel in einer Vorstufe oder einer Nachstufe des Matrixverarbeitungsabschnitts durchgeführt wird oder gleichzeitig mit der Farbumwandlungsverarbeitung des Matrixverarbeitungsabschnitts (52) durchgeführt wird.

4. Endoskopvorrichtung (1) nach Anspruch 3,
wobei das erste Band ein grünes Band ist und das zweite Band ein blaues Band ist.

**Revendications**

1. Appareil d'endoscope (1), comprenant :

des moyens d'éclairage (3) configurés pour réaliser un éclairage par une lumière d'éclairage d'une première bande, et réaliser des éclairages par une lumière d'éclairage d'une seconde bande un premier nombre de fois qui est deux fois ou plus, dans une période temporelle prédéterminée ;

des moyens de capture d'image (25) configurés pour capturer une image d'un objet éclairé par les moyens d'éclairage (3), et délivrer en sortie un premier signal de capture d'image basé sur l'éclairage de la lumière d'éclairage de la première bande, et des signaux de capture d'image basés sur des éclairages de la lumière d'éclairage de la seconde bande ; et

des moyens de calcul de brillance (44) configurés pour calculer une première brillance par traitement de matrice de conversion des couleurs en utilisant le premier signal de capture d'image et un deuxième signal de capture d'image basé sur un éclairage aux premières fois prédéterminées parmi le premier nombre de fois, et calculer une seconde brillance par traitement de matrice de conversion des couleurs en utilisant le premier signal de capture d'image et un troisième signal de capture d'image basé sur un éclairage à des fois autres que les premières fois prédéterminées parmi le premier nombre de fois,

**caractérisé par** :

des moyens de synthèse (45) configurés pour synthétiser et délivrer en sortie une image capturée par multiplication des premier et troisième signaux de capture d'image par un coefficient basé sur un rapport d'une valeur de différence d'une brillance cible et de la première brillance, et de la seconde brillance et par la suite synthèse du résultat avec les premier et deuxième signaux de capture d'image.

2. Appareil d'endoscope (1) selon la revendication 1,
dans lequel le premier nombre de fois est deux fois,
les moyens de calcul de brillance (44) sont configurés pour calculer la première brillance par traitement de matrice de conversion des couleurs en utilisant le premier signal de capture d'image et le deuxième signal de capture d'image basé sur un éclairage d'une première fois parmi le premier nombre de fois, et calculer la seconde brillance par traitement de matrice de conversion des couleurs en utilisant le premier signal de capture d'image et le troisième signal de capture d'image basé sur un éclairage d'une seconde fois parmi le premier nombre de fois.

3. Appareil d'endoscope (1) selon la revendication 2, comprenant en outre :

une section de traitement de matrice (52) configurée pour réaliser un traitement de conversion des couleurs correspondant à une entrée d'un système d'affichage qui réalise un affichage sur la base des premier à troisième signaux de capture d'image,

dans lequel une synthèse par les moyens de synthèse est réalisée au niveau d'un pré-étage ou d'un post-étage de la section de traitement de matrice, ou réalisée simultanément avec le traitement de conversion des couleurs de la section de traitement de matrice (52).

4. Appareil d'endoscope (1) selon la revendication 3,
dans lequel la première bande est une bande verte, et la seconde bande est une bande bleue.

# FIG.1

# FIG.2

# FIG.3

# FIG.4

IMAGE PROCESSING APPARATUS

104

SYNCHRONIZATION MEMORY — 40a

VIDEO SIGNAL

27 CONNECTOR

41 ANALOG PROCESSING SECTION

42 A/D CONVERTOR

43 DIGITAL PROCESSING SECTION

40 SYNCHRONIZATION CONTROL SECTION

45 SYNTHESIS PROCESSING SECTION

46 MATRIX PROCESSING SECTION

47 D/A CONVERTOR

5 MONITOR

100

BRIGHTNESS CALCULATION PROCESSING SECTION

52 MATRIX PROCESSING SECTION

44

49 LIGHT ADJUSTMENT CONTROL SECTION

105 CONTROL SECTION

LIGHT SOURCE APPARATUS

LIGHT SOURCE CONTROL SECTION — 106

34 DIAPHGRAM

113 ROTATION FILTER

32 NARROW BAND FILTER

31 LIGHT SOURCE (LAMP)

ILLUMINATING LIGHT

25 CCD
24
22
23

2
26
21

103

EP 2 671 498 B1

**FIG.5**

EP 2 671 498 B1

# FIG.6

# FIG.7

BRIGHT

DARK

| (a) | (b) | (c) | (d) |

(a): Bs1, Bs2, Bs3, Bs4, Bs5, Bs6, Bs7, ... , Bs94, Bs95, Bs96, Bs97, Bs98, Bs99, Bs100

(b): Bs10, Bs20, Bs9, Bs19, Bs28, Bs30, Bs8, ... , Bs93, Bs70, Bs73, Bs82, Bs92, Bs81, Bs91

(c): Bs10, Bs20, Bs9, Bs19, Bs28, Bs30, Bs8, ... , Bs93, Bs70, Bs73, Bs82, Bs92, Bs81, Bs91

Ash

Asd

EP 2 671 498 B1

# FIG.8

# FIG.9

# FIG.10

IMAGE PROCESSING APPARATUS 260

SYNCHRONIZATION MEMORY 40a

27 CONNECTOR — VIDEO SIGNAL

41 ANALOG PROCESSING SECTION

42 A/D CONVERTOR

63 WHITE BALANCE PROCESSING SECTION

43 DIGITAL PROCESSING SECTION

40 SYNCHRONIZATION CONTROL SECTION

48 AGC CIRCUIT

45 SYNTHESIS PROCESSING SECTION

62 ADJUSTMENT VALUE MEMORY

244 BRIGHTNESS CALCULATION PROCESSING SECTION

61 LIGHT ADJUSTMENT CONTROL SECTION

46 MATRIX PROCESSING SECTION

47 D/A CONVERTER

5 MONITOR

LIGHT SOURCE APPARATUS

34 DIAPHGRAM

33 ROTATION FILTER

32 NARROW BAND FILTER

31 LIGHT SOURCE (LAMP)

3

25 CCD
24
23
22
21
26
2

ILLUMINATING LIGHT

EP 2 671 498 B1

# FIG.11

FIG.12

# FIG.13

x

PRESENT INPUT FIELD

| a0 |
| a1 |
| a2 |
| a3 |

⋮

IMMEDIATELY PRECEDING
INPUT FIELD

| b0 |
| b1 |
| b2 |
| b3 |

⋮

OUTPUT DIRECTLY

GENERATE BY INTERPOLATION

OUTPUT FRAME

| p0=a0 |
| p1 |
| p2=a1 |
| p3 |
| p4=a2 |
| p5 |
| p6=a3 |
| p7 |

⋮

GENERATE INTERPOLATION DATA FOR RESPECTIVE PIXELS
BY THE FOLLOWING EXPRESSIONS.

$R(p3, x) = median\{R(a1, x), R(b1, x), R(a2, x)\}$

$G(p3, x) = median\{G(a1, x), G(b1, x), G(a2, x)\}$

$B(p3, x) = median\{B(a1, x), B(b1, x), B(a2, x)\}$

NOTE THAT median {a, b, c} REPRESENTS MEDIAN VALUE OF a, b, c.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010131620 A
- JP 2011185127 A
- JP 2011185128 A